# EUROPEAN PATENT APPLICATION

(11) **EP 0 955 378 A1**
(43) Date of publication of application: **10.11.1999**
(21) Application number: 98106224.3
(22) Date of filing: 06.04.1998
(51) Int. Cl.: C12Q 1/68

(54) **Method and kit for detecting a sequence variation in the human TGF-beta 1 gene**

(71) Applicant: Langdahl, Bente Lomholt, 8230 Aabyhoj (DK)
(72) Inventor: Langdahl, Bente Lomholt, 8230 Aabyhoj (DK)
(74) Representative: Nielsen, Leif

(57) **Abstract**

A method of detecting the presence or absence of the 713-8delC sequence variation in the human TGF-β1 gene is described, which method comprises provision of a sample comprising the TGF-β1 gene to be tested, or a relevant fragment thereof, subjecting the sample to a PCR amplification process using as upstream primer an oligonucleotide comprising a 15-100 nucleotides long section selected coherently from the following nucleotide sequence: 
*
3′ G TTT CGT ACC AAG TGA TGG CCG GCG
GCT CCA CTG GAC CGG TGG TAA GTA
CCG TAC TTG GCC GGA AAG GAC GAA
GAG TAC CGG TGG GGC GAC CTC TCC
CGG GTC GTA GAC 5′
 such as to comprise the bold A* (the A marked with an asterisk) nucleotide, and as downstream primer a 15-100 nucleotides long oligonucleotide selected such as to correspond to a coherent sequence section of the TGF-β1 gene, placed at least one nucleotide upstream the 713-8C or 713-8delC, respectively, position of the gene (cf. Figs. 1 and 2a,b of the drawings), then digesting the PCR amplification product(s) thus produced with a restriction enzyme recognizing and cleaving the following nucleotide sequence: 
CCA NNNNN TGG
GGT NNNNN ACC
 subjecting the digested product(s) to a restriction fragment length separation process and detecting whether a restriction fragment length corresponding to a cleavage of the PCR amplification product(s) is present or not.

## Description

The present invention relates to a method and kit for detecting a sequence variation in a gene. More particularly the invention relates to a method of detecting the presence or absence of a nucleotide sequense variation in the Transforming Growth Factor β1 gene (TGF-β1 gene). The invention also relates to particular oligonucleotide primers for use in PCR reactions performed e.g. in order to detect such nucleotide sequence variation. Also the invention concerns an oligonucleotide primer kit containing the particular primer(s).

### BACKGROUND OF THE INVENTION:

Osteoporosis is a common disease with increasing rate of occurrence, characterized by reduced bone mass and increased fracture risk which affects up to 40% of women and 12% of men at some point during life. It would therefore be of importance to improve the capability of predicting the risk of osteoporosis earlier in life and thereby the possibility of instituting relevant prevention treatments.

In todays clinical practice, the evaluation of the risk of developing osteoporosis in an individual is based on measurements of bone strength and family history of osteoporosis.

Twin and family studies have shown that up to 75% of the age-specific variation in bone density is genetically determined. Osteoporosis is an example of a complex trait with a non-Mendelian way of inheritance. The phenotype of the individual is determined by a combination of genetic, environmental, dietary and hormonal factors. In order to characterize responsible genes, we and others have looked for the impact of polymorphisms and mutations in "candidate genes" on bone mass, bone turnover and prevalence of osteoporosis. Identification of such genes involved in the patogenesis of osteoporosis would greatly improve the estimate of risk.

### POTENTIAL IMPACT OF TRANSFORMING GROWTH FACTOR β1 (TGF-β1):

TGF-β1 is released from bone matrix during bone resorption and subsequently activated by the low pH below the ruffled border of the resorbing osteoclasts. TGF-β1 has been implicated as a possible mediator of coupling between bone resorption and formation because:
1. TGF-β1 inhibits mature osteoclasts and proliferation of mononuclear osteoclast precursors in vitro,
2. the peptide inhibits fusion of mononuclear precursors into osteoclasts,
3. TGF-β1 has been found to stimulate proliferation or differentiation of preosteoblasts in vitro, and
4. bone matrix has the highest concentrations of TGF-β1 of all tissues.

The TGF-β1 gene consists of 7 exons, of which part of exon 5, exon 6 and exon 7 encode the active TGF-β1. TGF-β1 is produced and secreted as a propeptide of 390 amino acids by a variety of cell types, including osteoblasts. TGF-β1 is subsequently incorporated into bone matrix during matrix formation.

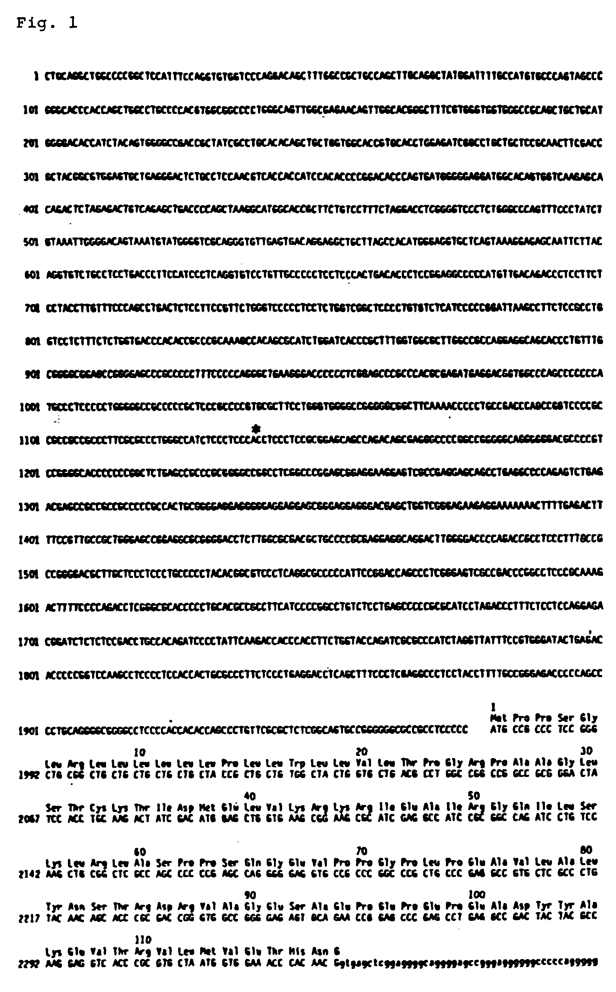

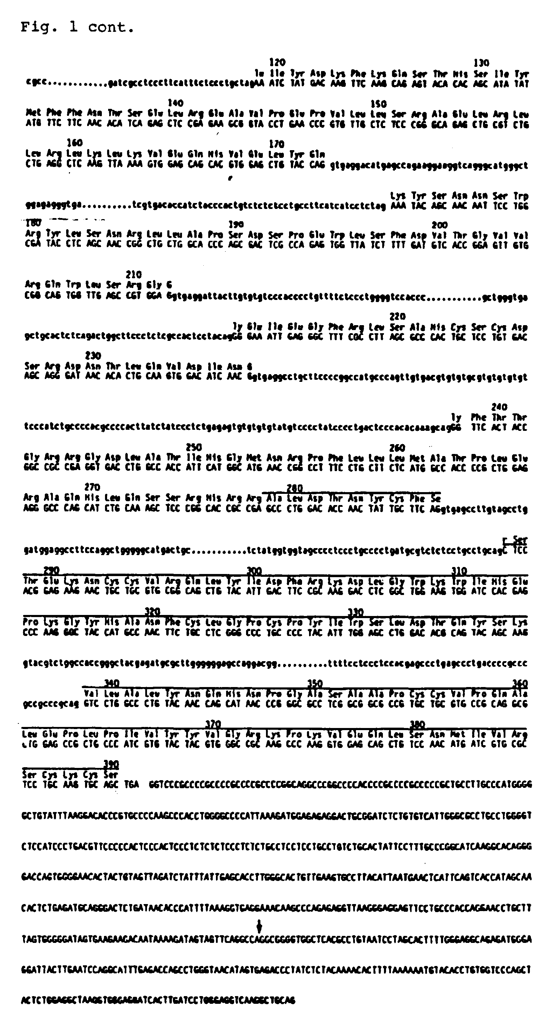

Derynck, R., Rhee, L., Chen, E.Y., and A. van Tilburg established in Nucl. Acids Res. 15 (7), pp. 3188-3189, (1987) the intron-exon structure and the complete base sequence of the human TGF-β1 precursor gene based on studies of two genomic DNA libraries, one derived from a normal fetal liver and one from the Calu-1 tumor cell line, cf. Fig. 1 of the attached drawings.

Based on polymerase chain reaction (PCR), polyamide gel electrophoresis (PGE) and semi-automated solid-phase dideoxynucleotide sequencing techniques Langdahl, B.L., Knudsen, J.Y. Jensen, H.K., Gregersen, N., and Eriksen, E.F. deseribe in Bone 20 (3), pp. 289-294, March 1997 the performance of a Single Stranded Conformation Polymorphism (SSCP) screening study of the TGF-β1 encoding gene derived from 161 osteoporotic women (i.e. having at least one low energy spinal fracture) and 131 normal women. The SSCP method reveals substitution(s) and/or deletion(s) of nucleic acid bases in the gene sequence examined.

Seven osteoporotic patients were found heterozygous for a cytosine to thymidine base substitution at position 76 in exon 5 (C⁷⁸⁸-T)(corresponding to position 788 in the TGF-β1 cDNA), resulting in a threonine to isoleucine amino acid shift at position 263 in the TGF-β1 propeptide (Thr²⁶³-Ile). Ten other osteoporotic patients had a one base deletion in the intron-4 sequence 8 bases prior to exon 5 (713-8delC, i.e. corresponding to position 713-8 in the TGF-β1 cDNA), that could influence splicing. Five normal women exhibited the C⁷⁸⁸-T sequence variant and two the 713-8delC variant.

The prevalence of 713-8delC was significantly higher in the osteoporotic group (X²=4.02, p<0.05). Osteoporotic patients with the 713-8delC variant had increased levels of bone alkaline phosphatase (p<0.05). If the osteoporotic patients with a Z-score below -1 in bone mineral density measurements of the lumber spine using dual-energy X-ray absorptiometri were examined separately, increased serum levels of bone alkaline phosphatase (p<0.05), increased urinary excretion of hydroxyproline (p<0.05) and reduced bone mass of the lumbar spine (p<0.05) were found in the patients with 713-8delC. No correlation to bone mass was demonstrated in the normal women, but 713-8delC was associated with increased serum levels of bone alkaline phosphatase (p<0.05).

The sequence variation 713-8delC in the TGF-β1 gene is more frequent in patients with osteoporosis compared to normal controls. The 713-8delC variant seems to be associated with very low bone mass in osteoporotic women with low bone mass, and with increased bone turnover in both osteoporotic and normal women.

The above study thus also revealed that the base sequence established by Derynck et al. (supra) for the intron 4 domaine of the TGF-β1 gene was apparently based on an abnormal gene comprising a C-deletion at the 713-8 position, whereas the normal gene in fact comprises a C-nucleotide at the 713-8 position (i.e. the position referring to the TGF-β1 cDNA).

### THE INVENTION:

Because the 713-8delC variation found in the above study could be a valuable genetic marker in the assessment of the risk for a given person to develop osteoporosis and the need for further research of the prevalence of this genetic variation and its occurence and impact in association with other osteoporotic risk factors there was a need for a quick and reliable method to detect this genetic 714-8delC variation.

The present invention provides a method which satisfies this need in all respects.

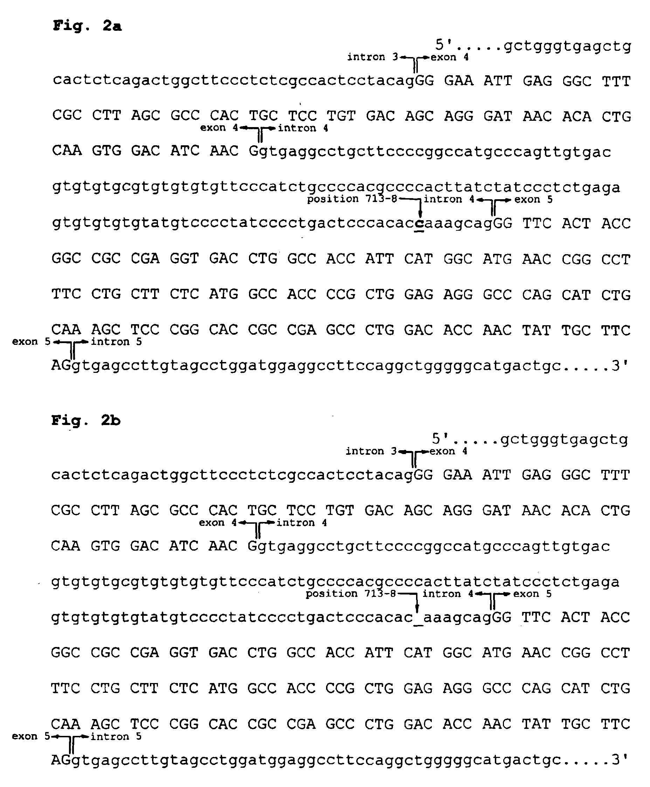

Thus, the present invention provides a method of detecting the presence or absence of the 713-8delC sequence variation in the human TGF-β1 gene, which method comprises provision of a sample comprising the TGF-β1 gene to be tested, or a relevant fragment thereof, subjecting the sample to a PCR amplification process using as upstream primer an oligonucleotide comprising a 15-100 nucleotides long section selected coherently from the following nucleotide sequence: 
*
3′ G TTT CGT ACC AAG TGA TGG CCG GCG
GCT CCA CTG GAC CGG TGG TAA GTA
CCG TAC TTG GCC GGA AAG GAC GAA
GAG TAC CGG TGG GGC GAC CTC TCC
CGG GTC GTA GAC 5′
 such as to comprise the bold A* (the A marked with an asterisk) nucleotide, and as downstream primer a 15-100 nucleotides long oligenucleotide selected such as to correspond to a coherent sequence section of the TGF-β1 gene, placed at least one nucleotide upstream the 713-8C or 713-8delC, respectively, position of the gene (cf. Figs. 1 and 2a,b of the drawings), then digesting the PCR amplification product(s) thus produced with a restriction enzyme recognizing and cleaving the following nucleotide sequence: 
CCA NNNNN TGG
GGT NNNNN ACC
 subjecting the digested product(s) to a restriction fragment length separation process and detecting whether a restriction fragment length(s) corresponding to a cleavage of the PCR amplification product(s) is (are) present or not.

In case the digested PCR amplification product(s) is (are) completely cleaved this indicates that the analyzed TGF-β1 gene is normal, i.e. it has a C-nucleotide at the 713-8 position in both strands of the gene.

In case the digested PCR amplification product(s) is (are) only partly cleaved this indicates that the analyzed TGF-β1 gene is a heterozygote 713-8delC+/- variant, i.e. it has a C-nucleotide at the 713-8 position in one of the strands of the gene and a deletion of the C-nucleotide in that position in the other strand of the gene.

In case the digested PCR amplification product(s) is (are) uncleaved this indicates that the analyzed TGF-β1 gene is a homozygote 713-8delC+/+ variant, i.e. it has a deletion of the C-nucleotide at the 713-8 position in both strands of the gene.

By a "relevant fragment" of the TGF-β1 gene is meant a coherent nucleotide sequence section comprising the 713-8C or 713-8delC position and extending at least 15, preferably at least 20, nucleotides downstream from that position into the exon 5 domain and at least 15, preferably at least 25, more preferably at least 50, and most preferably at least 80, nucleotides upstream from that position. Preferably, a "relevant fragment" of the gene comprises a 100 to 500 nucleotides section of the gene. Preferably, the fragment comprises the whole exon 4 and intron 4 domains and at least a part of the consecutive exon 5 domain of the gene.

In the nucleotide sequence: 
CCA NNNNN TGG
GGT NNNNN ACC
 N means any one of the four nucleotiodes A, C, G, and T.

The cleaving arrows shown mean that the cutting enzyme used may cut the sequence at the posotions indicated as well as at any position between the two terminal groups shown, i.e. between:

CCA TGG
and
GGT ACC

Preferably the upstream primer is 15-50, more preferably 15-40, yet more preferably 15-30, and most preferably 20-30, nucleotides long.

That the downstream primer is placed at least one nucleotide upstream the 713-8C or 713-8delC, respectively, position of the TGF-β1 gene means that the lowermost nucleotide at the 3' end af the primer is located at least one position upstream the 713-8C or 713-8delC, respectively, position of the gene. These positions refer to the TGF-β1 cDNA of the gene. However, the lowermost nucleotide at the 3' end of the primer is preferably at least located 25 nucleotide positions, more preferably at least 50 nucleotide positions, yet more preferably at least 100 nucleotide positions, and most preferably at least 150 nucleotide positions upstream the 713-8 position of the gene. Preferably the downstream primer is 15-50, more preferably 15-40, yet more preferably 15-30, and most preferably 20-30, nucleotides long.

The upstream and downstream primers are preferably selected such that the amplification products produced by the PCR method of the invention are at least 80 nucleotides long, preferably from 100 to 500 nucleotides long.

In addition to the particular primers used in the PCR amplification process also the usual other compounds and components used in such a process will be present such as the four mononucleotides dATP, dCTP, dGTP and dTTP, necessary salts like MgCl₂, buffer(s), and a polymerase enzyme like Taq polymerase, E-coli DNA-polymerase I, the Klenow fragmant of E-coli, T₄-DNA polymerase and other available suitable polymerases.

Preferred embodiments of the method are set forth in the attached claims 2-9.

The invention also concerns an oligonucleotide primer comprising a 15-100 nucleotides long section selected coherently from the nucleotide sequence: 
*
3′ G TTT CGT ACC AAG TGA TGG CCG GCG
GCT CCA CTG GAC CGG TGG TAA GTA
CCG TAC TTG GCC GGA AAG GAC GAA
GAG TAC CGG TGG GGC GAC CTC TCC
CGG GTC GTA GAC 5′
 such as to comprise the bold **A*** (the A marked with the asterisk).

Preferably, this oligonucleotide primer is 15-50, more preferably 15-40, yet more preferably 15-30, and most preferably 20-30, nucleotides long.

A particular preferred primer is:

*
3′ TT CGT ACC AAG TGA TGG CCG GCG 5′

Furthermore, the invention concerns a primer kit comprising an oligonucleotide primer as defined above (cf. claims 10-13) and another oligonucleotide primer comprising a 15-100 nucleotides long sequence corresponding to a coherent sequence section of the TGF-β1 gene placed at least one nucleotide upstream the 713-8C or 713-8delC, respectively, position of the TGF-β1 gene (cf. Figs. 1 and 2a,b of the drawings). The second oligonucleotide primer of the kit is preferably 15-50, more preferably 15-40, yet more preferably 15-30, and most preferably 20-30, nucleotides long.

A preferred primer kit comprises a primer which is: 
*
3′ TT CGT ACC AAG TGA TGG CCG GCG 5′
 and another primer which is
5' ATT GAG GGC TTT CGC CTT AGC GC 3'

The above primers may have other mismatching nucleotides incorporated in their sequence than the above indicated obligatory one (the bold **A** marked with the asterisk) provided such further mismatching nucleotide(s) do not impair or prevent their function as primers essentially and do not result in PCR products which when digested by the restriction enzyme lead to misleading restriction fragment lengths. Also on the same conditions the primers may be attached to non-complementary nucleotide fragments or particular markers or marker groups at their 5' ends.

Finally, the invention also concerns any DNA sequence comprising the sequence section: 
*
3′ TT CGT ACC AAG TGA TGG CCG GCG 5′
 as well as any primer pair comprising the DNA sequence. Preferably the DNA sequence comprises no more than 100 nucleotides, more preferably no more than 50 nucleotides, yet more preferably no more than 30 nucleotides, and at the 3' end it has preferably no more than 7 nucleotides exceeding beyond the bold A* (the A marked with an asterisk).

### DETAILED EXPLANATION OF THE THEORY ON WHICH THE INVENTION IS BASED:

Neither the 713-8C position of the TGF-β1 gene nor the 713-8delC variation thereof do themselves constitute natural restriction sites for any known endonuclease restriction enzyme. Therefore the particular idea was to investigate whether it would be possible by PCR technique to produce artificial nucleotiode amplification products which would be distinctive and discernible for one or more known endonuclease restriction enzymes depending on the fact whether the amplification product was derived from the normal 713-8C TGF-β1 gene or from the 713-8del C variation thereof.

The investigation instituted turned out to be successfull and the solution found will be explained in more details in the following on the basis of two particular primers and the particular restriction enzyme Van 91I.

The normal sequence of the intron 4 leading up to the exon 5 domain in the TGF-β1 gene is:
5' cccacaccaaagcagGG TTC ACT 3'
small letters: intron sequence, and capital letters: exon sequence, the underlined c represents the 713-8C position.

The sequence variant 713-8delC has a deletion at position 8 before the exon 5:
5' cccacac_aaagcagGG TTC ACT 3'

To detect the deletion the following primers are constructed:
- Downstream:: 5' ATT GAG GGC TTT CGC CTT AGC GC 3' (located in exon 4)
- Upstream:: 5' GCG GCC GGT AGT GAA CC**A** TGC TT 3' (located partly in intron 4 and partly in exon 5)

The Upstream primer has a mismatch (bold type **A**) when attached to the TGF-β1 gene at the appropriate portion:

cccacaccaaagcagGG TTC ACT ACC GGC CGC CGA GGT...
3′ TTCGTACC AAG TGA TGG CCG GCG 5′

The PCR-products are thus,
in the normal sequence:
...CC CAC ACC AAA GCA **T**GG TTC ACT ACC GGC CGC
in the variant sequence:
...CC CAC AC_ AAA GCA **T**GG TTC ACT ACC GGC CGC

(Bold type **T** introduced due to the presence of the mismatching bold type **A** in the upstream primer). And the final PCR amplification products, which will be double-stranded, are:

### Normal:

...CC CAC ACC AAA GCA **T**GG TTC ACT ACC GGC CGC
...GG GTG TGG TTT CGT **A**CC AAG TGA TGG CCG GCG

### Variant:

...CC CAC AC AAA GCA **T**GG TTC ACT ACC GGC CGC
...GG GTG TG TTT CGT **A**CC AAG TGA TGG CCG GCG

The restriction enzyme Van 91I recognizes and cuts the following nucleotide sequence: 
CCA NNNNN TGG
GGT NNNNN ACC
 where N can be any of the four nucleotides A, C, G, and T. Hence, the normal PCR product will be cleaved by the enzyme, the variant PCR product not:

### Normal:

clevage
...CC CAC ACC AAA GCA TGG TTC ACT ACC GGC CGC
...GG GTG TGG TTT CGT ACC AAG TGA TGG CCG GCG

### Variant:

no clevage
...CC CAC AC AAA GCA TGG TTC ACT ACC GGC CGC
...GG GTG TG TTT CGT ACC AAG TGA TGG CCG GCG

The invention will now be further explained with reference to the following example and the attached drawings in which:
- Fig. 1: shows the downstream strand nucleotide sequence of the toal TGF-β1 gene as established by Derynck et al. (supra),
- Fig. 2a: shows the downstream strand nucleotide sequence of the whole exon 4, intron 4 and exon 5 of the normal TGF-β1 gene as established by the present inventors, i.e. containing the normal c-nucleotide at the 713-8 position, and parts of the introns 3 and 5,
- Fig. 2b: shows the downstream strand nucleotide sequence of the whole exon 4, intron 4 and exon 5 of the 713-8delC variant of the TGF-β1 gene as established by the present inventors, i.e. having a deletion of the c-nucleotide at the 713-8 position, and parts of the introns 3 and 5,
- Fig. 3: is a photograph of an electrophoretic separation pattern in a 3% agarose gel of non-digested and digested PCR products produced by the method of the invention and showing seven separation lanes, the first one of which from the left being a marker lane, and
- Fig. 4: is a sketch of an electrophoretic separation pattern of another run in 3% agarose gel of non-digested and digested PCR products produced by the method of the invention and showing with greater clarity seven separation lanes, the first one of which from the left being a marker lane.

### EXAMPLE

Whole blood samples were collected from osteoporotic and normal women. DNA was isolated from whole blood leukocytes using a QIAamp Blood Kit® from QIAGEN®. The procedure used in obtaining purified DNA from the samples is described in the QIAamp Blood KIT and QIAamp Tissue Kit Handbook (December 1996), QIAGEN Inc., 28159 Avenue Stanford, Santa Clarita, CA 91355-1106, USA.

Polymerase chain reactions (PCR) were performed in a final volume of 50 µl using:
5 µl 10xPCR buffer (Perkin Elmer)
3 µl MgCl₂ 15 mM (Perkin Elmer)
0,1 µmol of each NTP (Perkin Elmer)
1,25 U Taq Polymerase (Perkin Elmer)
25 pmol upstream primer^{a)} and 25 pmol dowmstream primer^{b)}
2 µl DNA (100 ng/µl)
31 µl H₂O

a) upstream primer was:
5' GCG GCC GGT AGT GAA CC**A** TGC TT 3'
b) downstream primer was:
5' ATT GAG GGC TTT CGC CTT AGC GC 3'

The final PCR mixture was overlaid with 30 µl mineral oil. PCR was conducted in an automated Perkin Elmer/Cetus Thermal Cycler model 480 at the following conditions:

Initial denaturation: 10 min. at 95° C.
Denaturation at 95° C for 1 min.
35 Cycles: Annealing at 61° C for 1 min.
Primer extension at 74° C for 1 min.
Final primer extension: 8 min at 74° C.

### DIGESTION:

Samples of the PCR products produced were digested at 37° C for 2 hours using:
10 µl of the PCR product
0,5 µl Van 91I (5U/µl) (Boehringer Mannheim)
2 µl buffer B (Boehringer Mannheim)
8 µl H₂O

### ELECTROPHORESIS:

10 µl of non-digested and digested, respectively, PCR amplificatin products were placed in slots of a 3% agarose gel. As was 10 µl of a size marker solution from Boehringer-Mannheim designated "DNA Molecular Weight Marker V" and containing 22 DNA fragments of from 8 to 587 base-pairs in length spaced in groups of five different fragment base-pair lengths. The running buffer was a Tris-borate EDTA buffer (Tris-borate: 0.89 M, EDTA: 0.02 M), pH = 8.0, and electrohoretic separation was performed at 100V for 2 hours.

The electrophoretic separation pattern was visualized by staining the gel with ethidium bromide and visualization effected under UV-light.

### RESULTS AND DISCUSSION

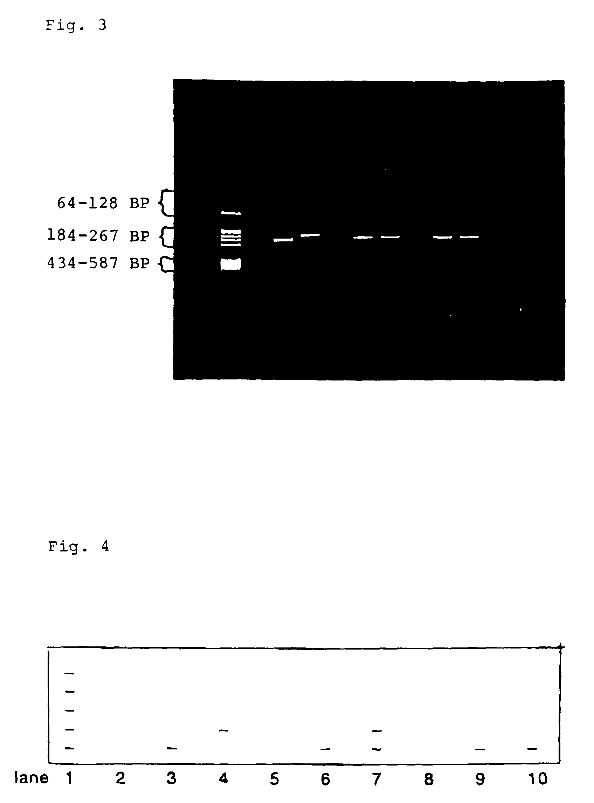

Fig. 3, which is a photograph of an electrophoretic separation pattern in a 3% agarose gel of non-digested and digested PCR amplification products produced by the method of the invention, shows seven separation lanes, which being from the left to the right as follows:
- lane 1:: size marker sample showing bands of DNA fragments of from 64 (uppermost) to 587 (lowermost) base-pairs in length, the group of bands in line horizontally with the bands in the other lanes represents DNA fragments of 267 (lowermost), 234, 213, 192, and 184 (uppermost, bands 192 and 184 being seen as one single broad band) base-pairs, supplied from Boehringer-Mannheim under the designation "DNA Molecular Weight Marker V"
- lane 3: : non-digested (i.e. uncleaved) normal TGF-β1 gene
- lane 4:: digested (i.e. cleaved) normal TGF-β1 gene
- lane 6:: non-digested heterozygote 713-8delC+/- TGF-β1 gene
- lane 7:: digested (partly cleaved) heterozygote 713-8delC+/- TGF-β1 gene
- lane 9:: non-digested homozygote 713-8delC+/+ TGF-β1 gene
- lane 10:: digested homozygote 713-8delC+/+ TGF-β1 gene
- lanes 2, 5, 8, and 11-13: being empty.

Lanes 3, 6, and 9 show that irrespective of whether the TGF-β1 gene is normal, a heterozygote or homozygote 713-8delC variant, the non-digested PCR amplification products produced by using a primer set according to the present invention will only produce one single band at substantially the same distance from the origin after subjection to electrophoretic separation; the band in lane 3, however, being retarded a minor distance for unaccountable experimental reasons.

Lane 4 shows that digested PCR amplification products produced from the normal TGF-β1 using a primer set according to the present invention will be completely cleaved (i.e. the PCR products of both strands of the gene cleaved) by the restriction enzyme used in the method of the invention leaving only one single, but displaced band (as compared to the same non-digested, uncleaved normal gene PCR products) after electrophoretic separation.

Lane 7 shows that digested PCR amplification products produced from the heterozygote 713-8delC+/- TGF-β1 gene using a primer set according to the present invention will be only partly cleaved (i.e. the PCR products of one strand of the gene cleaved, the PCR products of the other strand of the gene uncleaved) by the restriction enzyme used in the method of the invention leaving two distinct bands, one of which being displaced, the other being located at substantially the same position (as compared to the same non-digested, uncleaved gene PCR products) after electrophoretic separation.

Lane 10 shows that digested PCR amplification products produced from the homozygote 713-8delC+/+ TGF-β1 gene using a primer set according to the present invention will not be cleaved by the restriction enzyme used in the method of the invention leaving only one distinct band being located at substantially the same position (as compared to the same non-digested (uncleaved) gene PCR products) after electrophoretic separation.

Fig. 4, which is a sketch of another electrophoretic separation pattern run in similar manner in a 3% agarose gel as the electrophoretic separation patern shown in Fig. 3, of non-digested and digested PCR amplification products produced by the method of the invention, shows with greater clarity the same features as explained above for Fig. 3, except that another size marker sample is used and that the empty lanes 11-13 are absent from the sketch. Thus the following lanes are shown:
- lane 1:: size marker sample comprising DNA fragments mutually spaced by about 25 base-pairs
- lane 3:: non-digested (i.e. uncleaved) normal TGF-β1 gene
- lane 4:: digested (i.e. cleaved) normal TGF-β1 gene
- lane 6:: non-digested heterozygote 713-8delC+/- TGF-β1 gene
- lane 7:: digested (partly cleaved) heterozygote 713-8delC+/- TGF-β1 gene
- lane 9:: non-digested homozygote 713-8delC+/+ TGF-β1 gene
- lane 10:: digested homozygote 713-8delC+/+ TGF-β1 gene
- lanes 2, 5 and 8: being empty

### CONCLUDING REMARKS

Electrophoresis of digested PCR amplification products should always be run together with a non-digested (i.e. uncleaved) sample as a negative control.

After isolation of the DNA to be tested the PCR can be performed within 3-4 hours, digestion takes about 2 hours, and electrophoresis takes about another 2 hours. Thus, the total assay time takes about 7-8 hours. The number of samples run simultaneously depends on the PCR Thermocycler, but with one having a capacity of 96 samples per run it will be possible to genotype 95 + 1 negative control in one day.

The PCR runs very stably with the primers disclosed herein and the digesting assay results in 100 percent reproducible results.

Thus, having described the invention in both general and detailed terms the scope of the invention for which protection is applied, is defined in the attached claims. However, it should be understood that other features and modifications than those particularly described in this specification and claims, which are evident for the man skilled in the art and which will be advantageous in connection with the performance of the present invention, are also considered to be within the scope of the present invention.

## Claims

1. A method of detecting the presence or absence of the 713-8delC sequence variation in the human TGF-β1 gene, which method comprises provision of a sample comprising the TGF-β1 gene to be tested, or a relevant fragment thereof, subjecting the sample to a PCR amplification process using as upstream primer an oligonucleotide comprising a 15-100 nucleotides long section selected coherently from the following nucleotide sequence: 
*
3′ G TTT CGT ACC AAG TGA TGG CCG GCG
GCT CCA CTG GAC CGG TGG TAA GTA
CCG TAC TTG GCC GGA AAG GAC GAA
GAG TAC CGG TGG GGC GAC CTC TCC
CGG GTC GTA GAC 5′
 such as to comprise the bold **A*** (marked with the asterisk) nucleotide, and as downstream primer a 15-100 nucleotides long oligonucleotide selected such as to correspond to a coherent sequence section of the TGF-β1 gene, placed at least one nucleotide upstream the 713-8C or 713-8delC, respectively, position of the gene (cf. Figs. 1 and 2a,b of the drawings), then digesting the PCR amplification product(s) thus produced with a restriction enzyme recognizing and cleaving the following nucleotide sequence: 
CCA NNNNN TGG
GGT NNNNN ACC
 subjecting the digested product(s) to a restriction fragment length separation process and detecting whether a restriction fragment length(s) corresponding to a cleavage of the PCR amplification product(s) is (are) present or not.

2. A method according to claim 1, wherein the upstream primer is 15-50, more preferably 15-40, yet more preferably 15-30, and most preferably 20-30, nucleotides long.

3. A method according to claims 1 or 2, wherein the downstream primer is 15-50, more preferebly 15-40, yet more preferably 15-30, and most preferably 20-30, nucleotides long.

4. A method according to claim 1, wherein the upstream primer is: 
*
3′ TT CGT ACC AAG TGA TGG CCG GCG 5′
 and the downstream primer is:
5' ATT GAG GGC TTT CGC CTT AGC GC 3'

5. A method according to any of the preceding claims, wherein the upstream and downstream primers are selected such that the amplification products produced by the PCR method of the invention are at least 80 nucleotides long, preferably from 100 to 500 nucleotides long.

6. A method according to any of the preceding claims, wherein the upstream and/or the downstream primer have other mismatching nucleotides incorporated in their sequence than the above in claim 1 indicated obligatory one (the bold **A** marked with the asterisk*) provided such further mismatching nucleotide(s) does (do) not impair or prevent their function as primers substantially and does (do) not result in PCR amplification products which when digested by the restriction enzyme lead to misleading restriction fragment lengths, and optionally, on the same conditions the primers may be attached to non-complementary nucleotide fragments or particular markers or marker groups at their 5' ends.

7. A method according to any of the preceding claims, wherein the restriction enzyme is Van 91I, PflMI or AccB71.

8. A method according to any of the preceding claims wherein the PCR amplification is effected with Taq polymerase and the PCR conditions are approximately:
Initially: 10 min at 95°C
Cycles: (1 min at 95°C + 1 min at 61°C + 1 min at 74°C) x 35
Finally: 8 min at 74°C.

9. A method according to any of the preceding claims, wherein the restriction fragment length separation process is an electrophoretic separation process, preferably performed in agarose gel.

10. An oligonucleotide primer comprising a 15-100 nucleotides long section selected coherently from the following nucleotide sequence: 
*
3′ G TTT CGT ACC AAG TGA TGG CCG GCG
GCT CCA CTG GAC CGG TGG TAA GTA
CCG TAC TTG GCC GGA AAG GAC GAA
GAG TAC CGG TGG GGC GAC CTC TCC
CGG GTC GTA GAC 5′
 such as to comprise the bold **A*** (the A marked with the asterisk) nucleotide.

11. An oligonucleotide primer according to claim 10, said primer being 15-50, more preferably 15-40, yet more preferably 15-30, and most preferably 20-30, nucleotides long.

12. An oligonucleotide primer according to claim 10, said primer being:

*
3′ TT CGT ACC AAG TGA TGG CCG GCG 5′

13. An oligonucleotide primer according to any of claims 10-12, wherein said primer has other mismatching nucleotides incorporated in its sequence than the above in claim 10 indicated obligatory one (the bold **A** marked with the asterisk*) provided such further mismatching nucleotide(s) does (do) not impair or prevent its function as primer substantially and does (do) not result in PCR amplification products which when digested by a restriction enzyme lead to misleading restriction fragment lengths, and optionally, on the same conditions the said primer may be attached to non-complementary nucleotide fragments or particular markers or marker groups at its 5' end.

14. A primer kit comprising an oligonucleotide first primer according to any of claims 10-13 and another oligonucleotide second primer comprising a 15-100 nucleotides long sequense corresponding to a coherent sequence section of the TGF-β1 gene selected at least one nucleotide upstream the 713-8C or 713-8delC, respectively, position of the TGF-β1 gene (cf. Figs. 1 and 2a,b of the drawings).

15. A primer kit according to claim 14, wherein the first primer is 15-50, more preferably 15-40, yet more preferably 15-30, and most preferably 20-30, nucleotides long, and the second primer is 15-50, more preferebly 15-40, yet more preferably 15-30, and most preferably 20-30, nucleotides long.

16. A primer kit according to claim 14, wherein the first primer is: 
*
3′ TT CGT ACC AAG TGA TGG CCG GCG 5′
 and the second primer is:
5' ATT GAG GGC TTT CGC CTT AGC GC 3'.

17. A primer kit according to any of claims 14-16, wherein the first and/or the second primer have other mismatching nucleotides incorporated in their sequence than the above in claim 10 indicated obligatory one (the bold **A** marked with the asterisk*) provided such further mismatching nucleotide(s) does (do) not impair or prevent their function as primers substantially and does (do) not result in PCR amplification products which when digested by a restriction enzyme lead to misleading restriction fragment lengths, and optionally, on the same conditions the primers may be attached to non-complementary nucleotide fragments or particular markers or marker groups at their 5' ends.


(1) GENERAL INFORMATION:

(i) APPLICANT:
(A) NAME: Bente Lomholt Langdahl
(B) STREET: Sophus Bauditz Vej 55
(C) CITY: Aabyhoej
(E) COUNTRY: Denmark
(F) POSTAL CODE (ZIP): 8230

(ii) TITLE OF INVENTION: Method and kit for detecting a sequence
variation in a gene

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98106224.3

(2) INFORMATION FOR SEQ ID NO: 1:

(i) SEQUENCE CHARACTERISTICS:
(A) LENGTH: 109 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(x) PUBLICATION INFORMATION:
(A) AUTHORS: Langdahl, B.L.
Knudsen, J.Y.
Jensen, H.K.
Gregersen, N.
Eriksen, E.F.
(C) JOURNAL: Bone
(D) VOLUME: 20
(F) PAGES: 289-294
(G) DATE: March-1997
(x) PUBLICATION INFORMATION:
(A) AUTHORS: Derynck, R.
Rhee, L.
Chen, E.Y.
van Tilburg, A.
(C) JOURNAL: Nucleic Acids Res.
(D) VOLUME: 15
(F) PAGES: 3188-3189
(G) DATE: 1987

(xi) SEQUENCE DESCRIPTION: SEQ ID NO: 1:

CAGATGCTGG GCCCTCTCCA GCGGGGTGGC CATGAGAAGC AGGAAAGGCC GGTTCATGCC
60

ATGAATGGTG GCCAGGTCAC CTCGGCGGCC GGTAGTGAAC CATGCTTTG
109

(2) INFORMATION FOR SEQ ID NO: 2:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(x) PUBLICATION INFORMATION:
(A) AUTHORS: Langdahl, B.L.
Knudsen, J.Y.
Jensen, H.K.
Gregersen, N.
Eriksen, E.F.
(C) JOURNAL: Bone
(D) VOLUME: 20
(F) PAGES: 289-294
(G) DATE: March-1997
(x) PUBLICATION INFORMATION:
(A) AUTHORS: Derynck, R.
Rhee, L.
Chen, E.Y.
van Tilburg, A.
(C) JOURNAL: Nucleic Acids Res.
(D) VOLUME: 15
(F) PAGES: 3188-3189
(G) DATE: 1987

(xi) SEQUENCE DESCRIPTION: SEQ ID NO: 2:

GCGGCCGGTA GTGAACCATG CTT
23